# EUROPÄISCHE PATENTANMELDUNG

(11) **EP 4 049 583 A1**
(43) Veröffentlichungstag der Anmeldung: **31.08.2022**
(21) Anmeldenummer: 22157970.9
(22) Anmeldetag: 22.02.2022
(51) Int. Cl.: A61B 5/0245

(54) **HALTEVORRICHTUNG ZUR SCHAFTLOSEN BEFESTIGUNG VON EMG-/MYO- UND/ODER TS-ELEKTRODEN SOWIE DATENSATZ HIERFÜR**

(30) Priorität: 26.02.2021 DE 102021104732
(71) Anmelder: OT Supply GmbH, 86529 Schrobenhausen (DE)
(72) Erfinder: Dallmayer, Robert, 86529 Schrobenhausen (DE); Kurzhals, Christoph, 86529 Schrobenhausen (DE); Schaser, Werner, 86529 Schrobenhausen (DE)
(74) Vertreter: Kuhnen & Wacker Patent- und Rechtsanwaltsbüro PartG mbB

(57) **Zusammenfassung**

Die Erfindung betrifft eine Haltevorrichtung zur schaftlosen Befestigung von EMG-/Myo- und/oder TS-Elektroden an einer Körperoberfläche eines menschlichen oder tierischen Körpers mit einem Grundkörper (3) mit einer Halteeinrichtung (8), die dazu eingerichtet und ausgebildet ist, wenigstens eine Elektrodeneinrichtung (2) mittelbar oder unmittelbar bezüglich des Grundkörpers (3) zu haltern, wobei der Grundkörper (3) wenigstens eine Befestigungseinrichtung (4) zur mittelbaren oder unmittelbaren Befestigung eines Anbringungsmittels (5) der Haltevorrichtung (1) besitzt und das Anbringungsmittel (5) dazu eingerichtet und ausgebildet ist, den Grundkörper (3) bezüglich der Körperoberfläche zu fixieren.

## Beschreibung

Die Erfindung betrifft eine Haltevorrichtung zur schaftlosen Befestigung von EMG-/Myo- und/oder TS-Elektroden an Körperflächen eines menschlichen und/oder tierischen Körpers. Sie betrifft weiterhin einen diesbezüglichen Datensatz.

Im Bereich der Orthopädietechnik, insbesondere im Bereich der Prothetik, z. B. bei einer Versorgung eines Gliedmaßenstumpfes mit einer automatischen oder automatisierten Prothese, werden oftmals Elektroden eingesetzt, die auf einer Körperoberfläche eines menschlichen oder tierischen Körpers aufzubringen sind. Üblicherweise werden diese Elektroden mittels Klebepads oder dergleichen auf dem Körper befestigt. Insbesondere im Bereich von Trainings- und/oder Messgeräten, die dazu dienen, eine motorisch antreibbare Prothese mit den entsprechenden elektrischen Signalen versorgbar zu machen, indem diese eingelernt oder besonders trainiert werden, sind eine Vielzahl verschiedener Elektrodenbauarten auf dem Markt. Im Rahmen von Trainingsübungen ist es dabei erforderlich, dass derartige Elektroden reproduzierbar und relativ genau an bereits in vorhergehenden Trainingssitzungen ermittelten Positionen angebracht werden.

Des Weiteren ist es von großer Wichtigkeit, die Elektroden unter Umständen leicht repositionieren zu können und eine gute Kontaktierung zwischen Elektroden und Hautoberfläche auch im Bereich von Gliedmaßenstümpfen z. B. im Bereich von Narbengewebe herzustellen.

Insbesondere Klebeelektroden werden diesen Anforderungen nicht immer gerecht. Außerdem bedeutet es einen großen Aufwand, Befestigungsgurte und/oder andere Befestigungshilfsmittel vorrätig zu halten, um eine Vielzahl der auf dem Markt befindlichen hinsichtlich ihrer Bauart (Geometrie) unterschiedliche Elektroden, die auf eine Klebebefestigung verzichten, an dem Patientenkörper (menschlicher oder tierischer Körper) befestigen zu können.

Weiterhin besteht eine Notwendigkeit, gegebenenfalls mehrere Elektroden in direkter Nachbarschaft, d. h. eng beabstandet zueinander, zuverlässig auf der Körperoberfläche zu befestigen.

Eine weitere Anforderung ist, dass entsprechende Elektroden in hohem Maße reproduzierbar positionierbar sein sollen. Dies soll vor allem patientenspezifisch möglich sein.

Eine weitere Aufgabe der Erfindung ist es, zur Lösung der Aufgabe eine einfach herstellbare, insbesondere mit geringem Herstellaufwand herstellbare Haltevorrichtung anzugeben. Vorgenannte Aufgabe soll insbesondere im Bereich des 3D-Druckes lösbar sein.

Obige Aufgaben werden mit einer Haltevorrichtung mit den Merkmalen des Anspruchs 1 gelöst. Vorteilhafte Ausführungsformen sind in den Unteransprüchen angegeben. Hinsichtlich des Datensatzes werden die Aufgaben mit einem Datensatz mit den Merkmalen des Anspruchs 13 gelöst.

Erfindungsgemäß ist eine Haltevorrichtung zur schaftlosen Befestigung von EMG-/Myo- und/oder TS-Elektroden an einer Körperoberfläche eines menschlichen oder tierischen Körpers mit einem Grundkörper mit einer Halteeinrichtung ausgestattet, die dazu eingerichtet und ausgebildet ist, wenigstens eine Elektrodeneinrichtung mittelbar oder unmittelbar bezüglich des Grundkörpers zu halten, wobei der Grundkörper wenigstens eine Befestigungseinrichtung zur mittelbaren oder unmittelbaren Befestigung eines Anbringungsmittels der Haltevorrichtung besitzt und das Anbringungsmittel dazu eingerichtet und ausgebildet ist, den Grundkörper bezüglich der Körperoberfläche, insbesondere diese kontaktierend, zu fixieren.

Erfindungsgemäß ist eine Haltevorrichtung vorgesehen, welche einen Grundkörper hat, der zur Halterung der Elektrodeneinrichtung vorgesehen ist. Der Grundkörper selbst verfügt über Befestigungseinrichtungen, die es ermöglichen, den Grundköper mit seiner Adaptereinrichtung, z. B. mittels Gurten leicht und reproduzierbar an der Körperoberfläche, insbesondere diese kontaktierend, zu fixieren.

Mit einer solchen erfindungsgemäßen Vorrichtung entfällt für den Anwender (z. B. einen Physiotherapeuten) eine elektrodenspezifische Handhabung. Die Elektroden werden allesamt an dem Grundkörper befestigt, der dann mittels einheitlicher Anbringungsmittel in bequemer Art und Weise und immer mit der gleichen Vorgehensweise handhabbar sind. Dies erleichtert die Bedienung und erhöht die Reproduzierbarkeit und die Positioniergenauigkeit.

In einer bevorzugten Ausführungsform ist die Halteeinrichtung eine Ausnehmung im Grundkörper, die zur haltenden Aufnahme der Elektrodeneinrichtung wenigstens einer ersten Bauart ausgebildet ist.

Der Grundkörper ist in dieser Ausführungsform in besonders einfacher Art und Weise zur haltenden Aufnahme einer bestimmten Elektrodeneinrichtung (erster Bauart) ausgebildet. Zusätzliche Maßnahmen zur Befestigung sind nicht notwendig. Eine Aufnahmeausnehmung, die auf eine bestimmte Bauart von Elektrodeneinrichtungen ausgerichtet ist, ist zweckmäßiger Weise derart ausgestaltet, dass die am Markt am meisten auftretende Elektrodenbauform in dieser Ausnehmung aufnehmbar ist.

Weiterhin bevorzugt ist die Halteeinrichtung ein Adaptergehäuse, welches zur haltenden Aufnahme der Elektrodeneinrichtung wenigstens einer ersten Bauart ausgebildet ist und mit dem Grundkörper lösbar verbindbar ist.

Insbesondere zur haltenden Aufnahme verschiedenartiger Elektrodeneinrichtungen an ein und demselben Grundkörper ist es zweckmäßig, wenigstens ein Adaptergehäuse vorzusehen, welches auf die spezifischen Elektrodeneinrichtungen abgestimmt ist, und insbesondere in schneller und bevorzugt werkzeugloser Art und Weise mit dem Grundkörper lösbar verbindbar ist. So können eine Vielzahl unterschiedlicher Elektrodengeometrien in einfacher Art und Weise an ein und demselben Grundkörper befestigt werden.

Vorzugsweise ist die Halteeinrichtung eine Ausnehmung im Grundkörper zur haltenden Aufnahme der Elektrodeneinrichtung erster Bauart und es sind ein oder mehrere Adaptergehäuse vorhanden, die zum Halten von Elektrodeneinrichtungen zweiter oder weiterer Bauarten ausgebildet sind.

Mit obigen Maßnahmen wird die erfindungsgemäße Haltevorrichtung eine Universalhaltevorrichtung für eine Vielzahl von Elektrodeneinrichtungen, die allesamt mit einer Grundhaltevorrichtung befestigbar sind.

In einer bevorzugten Ausführungsform sind die Elektrodeneinrichtungen EMG-/Myo- und/oder TS-Elektroden.

Die erfindungsgemäße Haltevorrichtung eignet sich insbesondere für EMG/Myo- und /oder TS-Elektroden, welche in Bereich der Orthopädietechnik, insbesondere im Bereich der Prothetik häufig Anwendung finden.

In einer bevorzugten Ausführungsform besitzt der Grundkörper wenigstens eine Markierungsöffnung.

Die oben erwähnte Markierungsöffnung dient insbesondere dazu, eine fertig an oder auf der Körperoberfläche angebrachte, d. h. positionierte Elektrode in jeder Position zu markieren. Die Markierungsöffnungen dienen dazu, beispielsweise mittels geeigneter hautverträglicher Farbstifte Positionsmarkierungen für die Elektroden auf der Körperoberfläche anzubringen, um mittels solcher Positionsmarken eine Reproduzierbarkeit der Positionierung in zukünftigen Sitzungen zu erhöhen.

In einer weiteren bevorzugten Ausführungsform ist die Haltevorrichtung, insbesondere das Adaptergehäuse, steckbar, insbesondere rastend steckbar mit dem Grundkörper lösbar verbindbar.

Obige Maßnahmen dienen zu einer schnellen und problemlosen Verbindung zwischen dem Adaptergehäuse und dem Grundkörper.

Weiterhin bevorzugt ist eine erste Befestigungseinrichtung wenigstens ein Gurt- oder Riemenbügel.

Insbesondere bevorzugt ist eine zweite Befestigungseinrichtung wenigstens ein Gurt- oder Riemenbügel, der lösbar mit einer Gegenbefestigungseinrichtung des Grundkörpers verbindbar ist.

Als erste und zweite Befestigungseinrichtungen sind insbesondere Gurt- und/oder Riemenbügel vorgesehen, mit denen mittels geeigneter Gurte und/oder Riemen oder Bänder, die durch Gurt- und/oder Riemenbügel gefädelt werden können, der Grundkörper auf der Körperoberfläche befestigbar ist.

In einer weiteren bevorzugten Ausführungsform ist die erste Befestigungseinrichtung, insbesondere der erste Gurt- oder Riemenbügel eines ersten Grundkörpers in die Gegenbefestigungseinrichtung eines zweiten Grundkörpers einsteckbar, so dass die Grundkörper miteinander verbindbar, insbesondere gelenkig verbindbar, sind.

In bevorzugter Art und Weise ist wenigstens ein Gurt- oder Riemenbügel lösbar mit einer am Grundkörper angebrachten Gegenbefestigungseinrichtung verbindbar. Dies ermöglicht es, eine Mehrzahl von Grundkörpern kettenartig/bandartig aneinander zu binden und somit eine eng benachbarte Positionierung der Elektroden unter vorgegebenen Abstand zu verwirklichen.

Mit einer derartigen Ausgestaltung ist es durch Einstecken eines Gurt- oder Riemenbügels in die entsprechende Gegenbefestigungseinrichtung auch schnell möglich, einen zuletzt in einer verketteten Art und Weise verwendeten Grundkörper wieder als Einzelgrundkörper zu verwenden.

Obige Maßnahmen erhöhen somit die flexible Einsetzbarkeit der erfindungsgemäßen Haltevorrichtung.

Im Folgenden wird die Erfindung anhand der Figuren beispielhaft näher erläutert. Es zeigen:
Figur 1: eine perspektivische Unteransicht auf eine erste Ausführungsform der erfindungsgemäßen Haltevorrichtung;
Figur 2: eine zweite perspektivische Unteransicht auf die erste Ausführungsform der erfindungsgemäßen Haltevorrichtung;
Figur 3: eine perspektivische Draufsicht auf die erste Ausführungsform der erfindungsgemäßen Haltevorrichtung;
Figur 4: eine perspektivische Ansicht auf die erste Ausführungsform der erfindungsgemäßen Haltevorrichtung mit einem Haltegurt;
Figur 5: eine Vielzahl von unterschiedlichen Ausführungsformen von Adaptergehäusen zur Aufnahme unterschiedlicher Elektroden, jeweils in Draufsicht;
Figur 6: eine perspektivische Ansicht eines Adaptergehäuses gemäß der Erfindung mit einer montierten Elektrodeneinrichtung;
Figur 7: eine weitere Ausführungsform eines Adaptergehäuses mit einem Adapterring und einer Einsetzplatte.
Figur 8: eine verkettete Anordnung zweier Adaptergehäuse gemäß der Erfindung in einem Querschnitt.

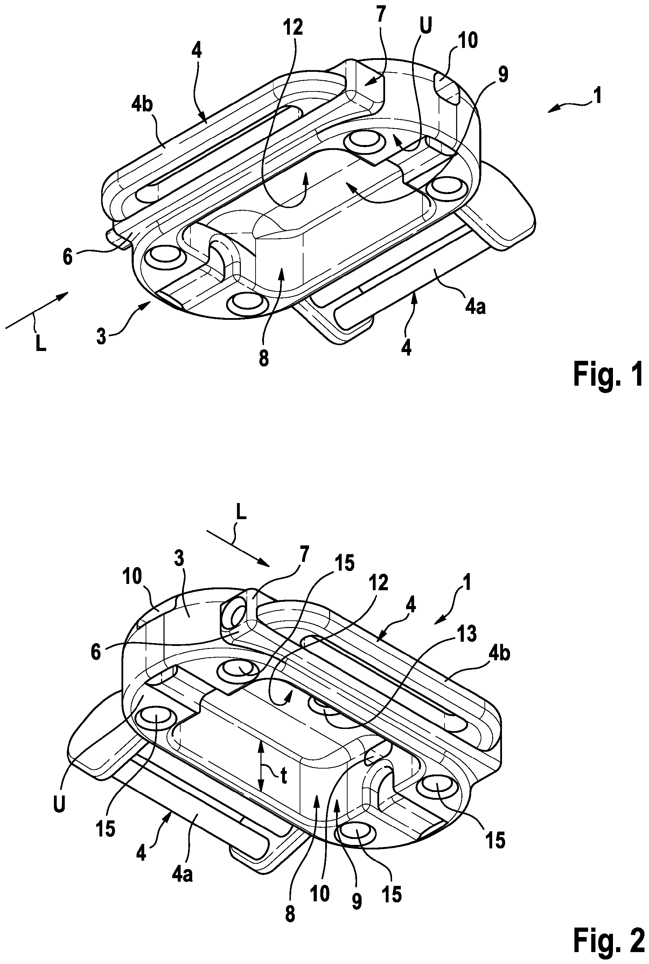

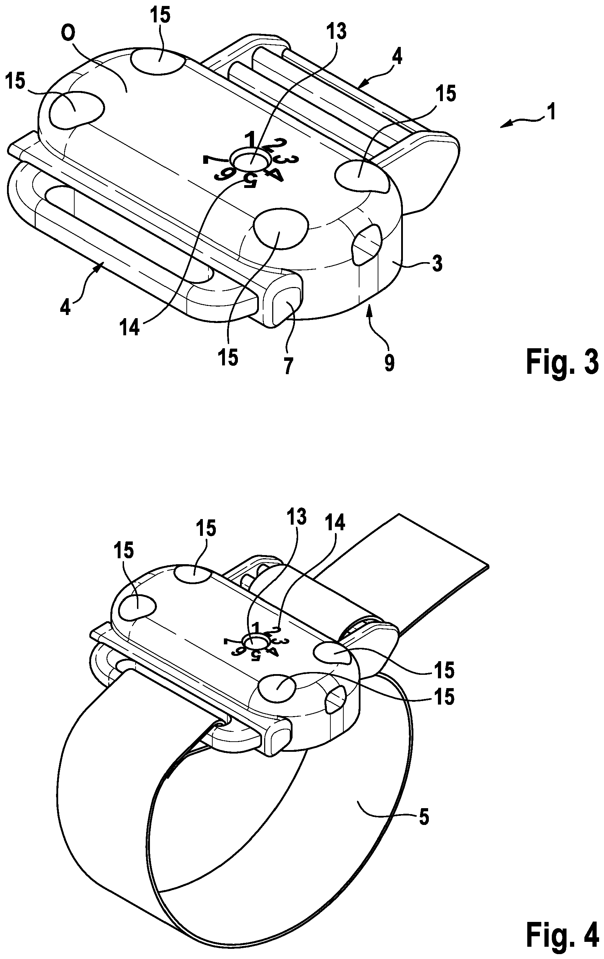

Eine erste Ausfiihrungsform der erfindungsgemäßen Haltevorrichtung 1 zeigen Figuren 1 bis 3, wobei die Figuren 1 und 2 jeweils perspektivische Unteransichten auf diese Ausführungsform sind und Figur 3 eine perspektivische Draufsicht auf diese Ausführungsform ist.

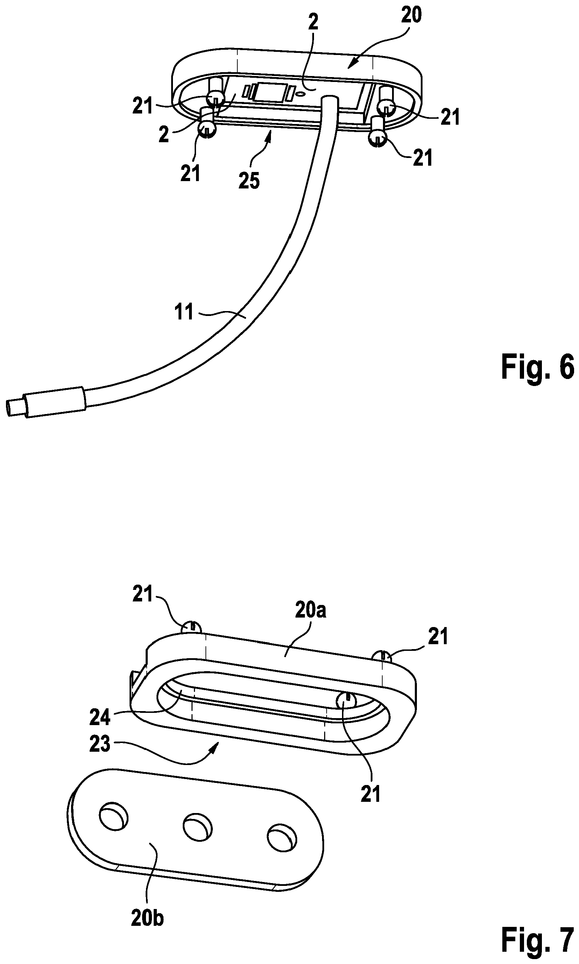

Die erfindungsgemäße Haltevorrichtung 1 ist zur Halterung einer Elektrodeneinrichtung 2 (vgl. Figur 6), z. B. einer EMG-MYO- und/oder TS-Elektrodeneinrichtung ausgebildet. Die Elektrodeneinrichtungen 2 sollen an Körperoberflächen (nicht gezeigt) eines menschlichen oder tierischen Körpers, insbesondere im Bereich eines Gliedmaßenstumpfes eingesetzt werden, um elektrische Impulse (Muskelreize) aufnehmen zu können.

Die Haltevorrichtung 1 besitzt als Halteeinrichtung einen Grundkörper 3. Am Grundkörper 3 ist eine Befestigungseinrichtung 4 vorgesehen, welche zur mittelbaren oder unmittelbaren Befestigung eines Anbringungsmittels 5 (vgl. Figur 4) vorgesehen ist. Das Anbringungsmittel 5 ist dazu eingerichtet und ausgebildet, den Grundkörper 3 bezüglich der Körperoberfläche zu fixieren. Das Anbringungsmittel 5 ist beispielsweise ein Riemen oder Gurt. Die Befestigungseinrichtung 4 kann in zweckmäßiger Art und Weise ein Gurt- oder Riemenbügel erster Art 4a und ein Gurt- oder Riemenbügel zweiter Art 4b sein. Der Gurt- oder Riemenbügel erster Art 4a ist dabei einstückig, d. h. integral mit dem Grundkörper 3 ausgebildet. Der Gurt- oder Riemenbügel zweiter Art 4b ist als Einsteckbügel ausgebildet, der lösbar in eine Einsteckschiene 6 in einer Längsrichtung L mit dem Grundkörper 3 lösbar verbindbar ist. Die Einsteckschiene 6 wirkt somit als Gegenbefestigungseinrichtung 7 für den Gurt- oder Riemenbügel zweiter Art 4b.

Die Einsteckschiene 6 ist somit ein spezielles Ausführungsbeispiel für eine allgemein lösbar mit dem Gurt- oder Riemenbügel zweiter Art 4b zusammenwirkenden Gegenbefestigungseinrichtung 7, welche zum Zwecke der vorliegenden Beschreibung mit dem Bezugszeichen 7 versehen wird.

Die Haltevorrichtung 1 weist eine Halteeinrichtung 8 auf, welche dazu eingerichtet und ausgebildet ist, wenigstens eine Elektrodeneinrichtung 2 bezüglich des Grundkörpers 3 zu halten.

In der Ausführungsform gemäß der Figuren 1, 2 und 3 ist die Halteeinrichtung 8 eine Ausnehmung 9, welche auf einer im bestimmungsgemäßen Gebrauch der Körperoberfläche zugewandten Unterseite U des Grundkörpers 3 vorgesehen ist. Die Ausnehmung 9 ist als taschenartige Ausnehmung 9 ausgebildet und hinsichtlich ihrer Tiefe t und ihrer Umfangskontur an eine bestimmte Bauart einer oder mehrerer auf dem Markt befindlichen Elektrodeneinrichtung 2 angepasst, so dass die Ausnehmung 9 als Aufnahme für die Elektrodeneinrichtung 2 der bestimmten Bauart dienen kann. Die Elektrodeneinrichtung 2 kann beispielsweise in die Ausnehmung 9 eingesenkt werden. Um abgehende elektrische Verbindungskabel 11 von der Elektrodeneinrichtung 2 aus dem Grundkörper 3 herausführen zu können, sind beispielsweise Durchführkanäle 10 vorgesehen, welche das Innere der Ausnehmung 9 mit der Außenumgebung verbinden. Durch die Durchführkanäle 10 können Verbindungskabel 11 (vgl. Figur 6) der Elektrodeneinrichtung 2 geführt werden.

Die Verbindungskabel 11 sind in den Figuren 1 bis 3 nicht dargestellt. Zur erläuternden Darstellung wird hierzu auf die Darstellung der Verbindungskabel 11 in Figur 6 verwiesen.

Somit kann mit dem Grundkörper 3 alleine eine Elektrodeneinrichtung 2 einer ersten Bauart von vielen Elektrodeneinrichtungen 2 unterschiedlicher Bauart, die sich auf dem Markt befinden, gehalten werden. Mittels der Befestigungseinrichtung 4 kann der Grundkörper 3, in dem die Elektrodeneinrichtung 2 eingesetzt ist, in einfacher Art und Weise bezüglich der Körperoberfläche des menschlichen oder tierischen Körpers positioniert und dort befestigt werden.

In zweckmäßiger Art und Weise besitzt eine Bodenwandung 12 der Ausnehmung 9 eine Durchtrittsöffnung 13, durch die bei einer eingesetzten Elektrodeneinrichtung 2 von einer Oberseite O, d. h. frei zugänglich mittels eines Werkzeuge, z. B. eines Schraubenziehers, z. B. ein Einstellpotentiometer der Elektrodeneinrichtung 2 erreicht werden kann. Zweckmäßiger Weise ist um die Durchtrittsöffnung 13 herum eine Skala 14 angebracht, welche einer Skala 14 auf der Elektrodenaußenseite (nicht gezeigt) entspricht. Hierdurch können in einfacher Art und Weise Einstellungen an den Elektrodeneinrichtungen 2 vorgenommen werden, obwohl diese optisch verdeckt in dem Grundkörper 3 aufgenommen sind.

Bei dem Einsatz von Elektrodeneinrichtungen 2, z. B. EMG-/MYO- und/oder TS-Elektroden ist es insbesondere für Trainingszwecke, zum Anpassen einer möglichen Prothese an einen Gliedmaßenstumpf besonders essentiell, eine Wiederholgenauigkeit der Positionierung der Elektrodeneinrichtungen 2 über mehrere Trainingssitzungen eines Patienten hinweg gut reproduzierbar auszugestalten. Dies verringert den Einrichtungsaufwand am Beginn einer neuen Trainingssitzung erheblich und lässt Trainingsentwicklungen und Trainingserfolge und/oder Misserfolge reproduzierbarer und genauer erkennen.

Um eine reproduzierbare Positionierung der Elektrodeneinrichtungen 2 über mehrere Trainingssitzungen hinweg zu gewährleisten, sind beispielsweise Markierungsöffnungen 15 vorgesehen, die im Grundkörper 3 eingelassen sind und es beispielsweise mit einem Markierungsstift (mit hautverträglicher Tinte) ermöglichen, eine einmal positionierte Elektrodeneinrichtung 2 durch die Markierungsöffnungen 15 hindurch auf der Hautoberfläche (Körperoberfläche) des Patienten zu markieren. Somit kann in einfacher Art und Weise die Positionierung der Elektroden festgehalten werden.

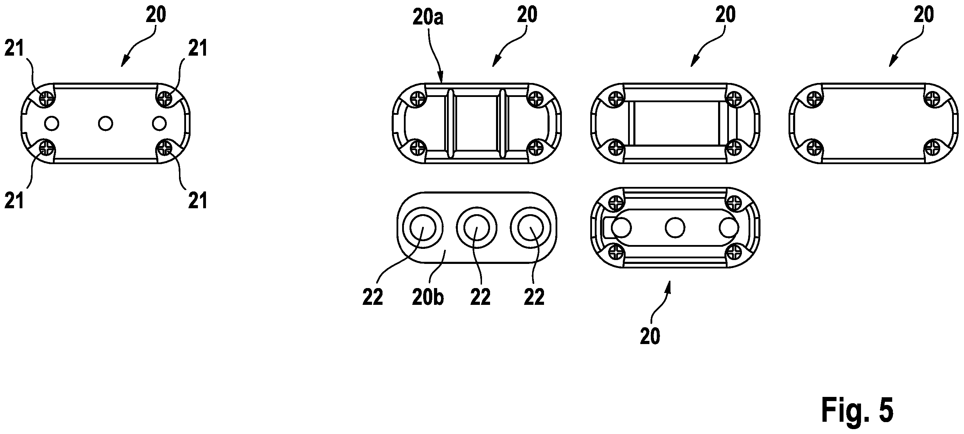

Zur Erhöhung der Flexibilität der erfindungsgemäßen Haltevorrichtung 1, insbesondere um eine Vielzahl von auf dem Markt befindlichen Elektrodeneinrichtungen 2 unterschiedlichster Bauart mit ein und derselben erfindungsgemäßen Haltevorrichtung 1 zu halten und bezüglich der Körperoberfläche des menschlichen oder tierischen Körpers positionieren zu können, besitzt die erfindungsgemäße Haltevorrichtung 1 zweckmäßiger Weise eine Vielzahl von Adaptergehäusen 20 (vgl. Figur 5). Die dort dargestellten Adaptergehäuse 20 sind jeweils auf eine bestimmte Elektrodeneinrichtung 2 abgestimmt und in ihrer Geometrie derart ausgebildet, dass eine bestimmte Elektrodeneinrichtung 2 mechanisch im Adaptergehäuse 20 gehalten werden kann.

Die Adaptergehäuse 20, die zur erfindungsgemäßen Haltevorrichtung 1 passen, besitzen zur Anbindung der Adaptergehäuse 20 an den Grundkörper 3 beispielsweise Stifte 21, welche derart ausgebildet und angeordnet sind, dass sie von der Unterseite U her in die Markierungsöffnungen 15 einsteckbar sind.

Ein Adaptergehäuse 20 kann beispielsweise, wie dargestellt in Figur 5, einen Adapterrahmen 20a besitzen, in den eine Adapterplatte 20b einsetzbar ist, wobei der Adapterrahmen 20a und die Adapterplatte 20b zusammen das Adaptergehäuse 20 bilden. Die Adapterplatte 20b weist dabei Öffnungen 22 zur Aufnahme von beispielsweise Einzelelektroden auf.

Eine solche Konstruktion des Adaptergehäuses 20 gebildet aus dem Adapterrahmen 20a und der Adapterplatte 20b ist etwas detaillierter in der Figur 7 dargestellt.

Der dort gezeigte Adapterrahmen 20a besitzt die Stifte 21 zum Zusammenwirken und Einstecken in die Markierungsöffnungen 15 des Grundkörpers 3. Die Adapterplatte 20b der Ausführungsform gemäß Figur 7 ist derart ausgebildet, dass sie in eine Einstecköffnung 23 des Adapterrahmens 20a passt. Elektrodeneinrichtungen 2, die zwischen der Adapterplatte 20b und dem Adapterrahmen 20a anzuordnen sind, werden beispielsweise mittels eines umlaufenden Vorsprungs 24 zwischen dem Adapterrahmen 20a und der Adapterplatte 20b gehalten.

Eine weitere Ausführungsform eines Adaptergehäuses 20 mit einer eingesetzten Elektrodeneinrichtung 2 zeigt Figur 6. Das dort gezeigte Adaptergehäuse 20 ist einstückig und besitzt zur Verbindung mit dem Grundkörper 3 die Stifte 21. Der Adapterrahmen 20 ist zur Aufnahme der gezeigten Elektrodeneinrichtung 2 explizit ausgebildet und besitzt eine geeignete Adapterrahmenöffnung 25, durch die das Verbindungskabel 11 der Elektrodeneinrichtung 2 hindurchgreifen kann. Das Verbindungskabel 11 im gezeigten Bespiel gemäß Figur 6 wird dann beim Einsetzen des Adapterrahmens 20a in den Grundkörper 3 bzw. beim Verbinden des Adapterrahmens 20a mit dem Grundkörper 3 durch die entsprechenden Durchführkanäle 10 geführt und kann somit in einfacher Art und Weise aus den Grundkörper 3 herausgeführt werden.

Besonders vorteilhaft bei der vorliegenden Erfindung ist, dass durch geeignete Ausbildung der Befestigungseinrichtungen 4 mit einer lösbar befestigbaren Befestigungseinrichtung zweiter Art 4b in einfacher Art und Weise ein Aneinanderketten von mehreren Grundkörpern 3 möglich ist, in dem beispielsweise die Befestigungseinrichtung erster Art 4a dazu eingerichtet und ausgebildet ist, in die Gegenbefestigungseinrichtung 7, im Beispiel gemäß Figuren 1 bis 3 also in die Einsteckschiene 6 einclipsbar oder einschiebbar ist.

Mit dieser Vorgehensweise gelingt es direkt, d. h. ohne Zwischenschaltung von Gurten- oder Riemenverbindungen zwei Grundkörper 3 aneinanderzuketten und gelenkig miteinander zu verbinden. Hierdurch sind insbesondere enge Elektrodenabstände in einfacher Art und Weise realisierbar, um beispielsweise nahe beieinanderliegende Signalquellen auf der Körperoberfläche des menschlichen oder tierischen Körpers erfassen zu können.

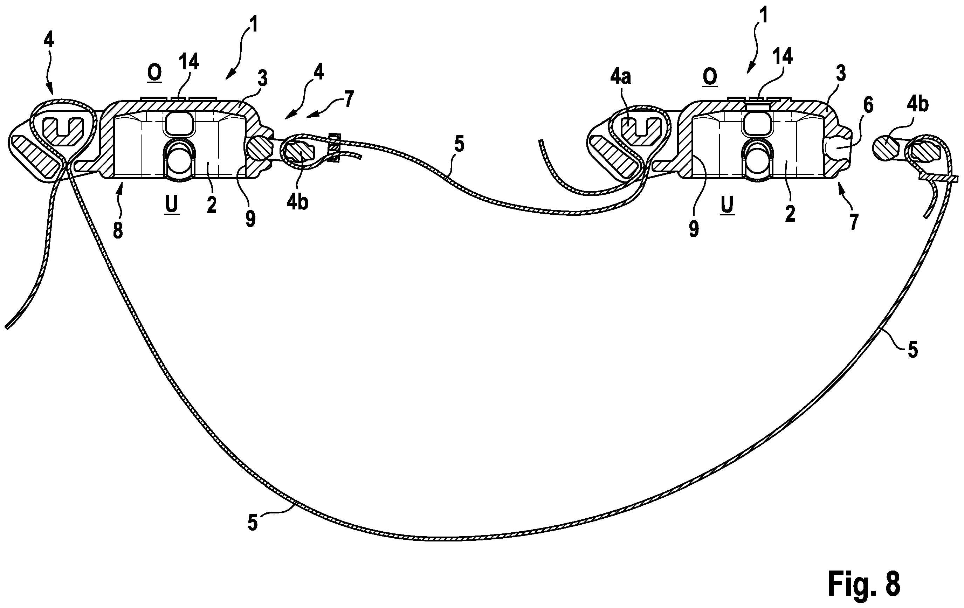

Im Rahmen der Figur 8 ist im Querschnitt eine Aneinanderkettung zweier Haltervorrichtungen 1 mittels eines Riemens gezeigt, der als Anbringungsmittel 5 wirkt. Freie Enden der Haltevorrichtungen 1 sind mit einem weiteren Riemenbügel, der ebenfalls als Anbringungsmittel 5 wirkt, verbunden. Eine Aneinanderkettung zweier oder mehrerer Haltevorrichtungen 1 mittels Riemen hat den Vorteil, dass die Haltevorrichtungen 1 am Körper bzw. auf der Körperoberfläche des menschlichen oder tierischen Körpers zueinander verschränkt angeordnet werden können und somit eine verbesserte Kontaktierung der Elektrodeneinrichtungen 2 mit der Hautoberflächen des menschlichen oder tierischen Körpers stattfinden kann. Die Verschränkung ist durch eine Verdrillung des Riemens zwischen den beiden Haltevorrichtungen 1 ermöglicht.

Bei der erfindungsgemäßen Haltevorrichtung 1 ist es somit von besonderem Vorteil, dass mit einer einfach gearteten Vorrichtung und durch das Vorhalten eines Grundkörpers 3 und einer verschiedenen Anzahl von Adaptergehäusen 20 mit geringem Aufwand eine Vielzahl von verschiedenen auf dem Markt befindlichen Elektrodeneinrichtungen 2 einheitlich und reproduzierbar für Trainingszwecke einsetzbar gemacht werden. Solche Trainingszwecke und solche Elektrodenpositionierungen sind insbesondere vor der Entwicklung einer patientenindividuellen Prothese, also z. B. wenn noch kein Halteschaft für den Patientenstumpf vorliegt, sehr wichtig. Dies z. B. dafür, um für einen bestimmten Schaft patientenindividuell die bestmöglichen Orte und Positionen für Aufnahmeelektroden feststellen zu können. Für diese Ermittlungsarbeit sind leicht bezüglich der Körperoberfläche verschiebbare aber reproduzierbar positionierbare Elektrodenhalterungen gemäß der Erfindung besonders vorteilhaft.

### Bezugszeichenliste

- 1: Haltevorrichtung
- 2: Elektrodeneinrichtung
- 3: Grundkörper
- 4: Befestigungseinrichtung
- 4a: Gurt- oder Riemenbügel erster Art
- 4b: Gurt- oder Riemenbügel zweiter Art
- 5: Anbringungsmittel
- 6: Einsteckschiene
- 7: Gegenbefestigungseinrichtung
- 8: Halteeinrichtung
- 9: Ausnehmung
- 10: Durchführkanäle
- 11: Verbindungskabel
- 12: Bodenwandung
- 13: Durchtrittsöffnung
- 14: Skala
- 15: Markierungsöffnung
- 20: Adaptergehäuse
- 20a: Adapterrahmen
- 20b: Adapterplatte
- 21: Stifte
- 23: Einstecköffnung
- 24: Vorsprung
- 25: A-Rahmenöffnung
- L: Längsrichtung
- t: Tiefe
- U: Unterseite
- O: Oberseite

## Patentansprüche

1. Haltevorrichtung zur schaftlosen Befestigung von EMG-/Myo- und/oder TS-Elektroden an einer Körperoberfläche eines menschlichen oder tierischen Körpers mit einem Grundkörper (3) mit einer Halteeinrichtung (8), die dazu eingerichtet und ausgebildet ist, wenigstens eine Elektrodeneinrichtung (2) mittelbar oder unmittelbar bezüglich des Grundkörpers (3) zu haltern, wobei der Grundkörper (3) wenigstens eine Befestigungseinrichtung (4) zur mittelbaren oder unmittelbaren Befestigung eines Anbringungsmittels (5) der Haltevorrichtung (1) besitzt und das Anbringungsmittel (5) dazu eingerichtet und ausgebildet ist, den Grundkörper (3) bezüglich der Körperoberfläche zu fixieren.

2. Haltevorrichtung nach Anspruch 1, **dadurch gekennzeichnet, dass** die Halteeinrichtung (8) eine Ausnehmung (9) im Grundkörper (3) ist, die zur haltenden Aufnahme der Elektrodeneinrichtung (2) wenigstens einer ersten Bauart ausgebildet ist.

3. Haltevorrichtung nach Anspruch 1 oder 2, **dadurch gekennzeichnet, dass** die Halteeinrichtung (8) ein Adaptergehäuse (20) ist, welches zur haltenden Aufnahme der Elektrodeneinrichtung (2) wenigstens einer ersten Bauart ausgebildet ist und mit dem Grundkörper (3) lösbar verbindbar ist.

4. Haltevorrichtung nach einem der vorangegangenen Ansprüche, **dadurch gekennzeichnet, dass** die Halteeinrichtung (8) eine Ausnehmung (9) im Grundkörper (3) zur haltenden Aufnahme der Elektrodeneinrichtung (2) erster Bauart ist und ein oder mehrere Adaptergehäuse (20) vorhanden sind, die zum Halten von Elektrodeneinrichtungen (2) zweiter oder weiterer Bauarten ausgebildet sind.

5. Haltevorrichtung nach einem der vorangegangenen Ansprüche, **dadurch gekennzeichnet, dass** die Elektrodeneinrichtungen (2) EMG-/Myo- und/oder TS-Elektroden sind.

6. Haltevorrichtung nach einem der vorangegangenen Ansprüche, **dadurch gekennzeichnet, dass** der Grundkörper (3) wenigstens eine Markierungsöffnung (15) besitzt.

7. Haltevorrichtung nach einem der vorangegangenen Ansprüche, **dadurch gekennzeichnet, dass** die Halteeinrichtung (8), insbesondere das Adaptergehäuse (20), steckbar, insbesondere rastend steckbar mit dem Grundkörper (3) lösbar verbindbar ist.

8. Haltevorrichtung nach einem der vorangegangenen Ansprüche, **dadurch gekennzeichnet, dass** eine erste Befestigungseinrichtung (4) wenigstens ein Gurt- oder Riemenbügel (4a; 4b) ist.

9. Haltevorrichtung nach einem der vorangegangenen Ansprüche, **dadurch gekennzeichnet, dass** eine zweite Befestigungseinrichtung (4) wenigstens ein Gurt- oder Riemenbügel (4b) ist, der lösbar mit einer Gegenbefestigungseinrichtung (7) des Grundkörpers (3) verbindbar ist.

10. Haltevorrichtung nach einem der vorangegangenen Ansprüche, **dadurch gekennzeichnet, dass** die erste Befestigungseinrichtung (4), insbesondere der Gurt- oder Riemenbügel erster Art (4a) eines ersten Grundkörpers (3) in die Gegenbefestigungseinrichtung (7) eines zweiten Grundkörpers (3) einsteckbar ist, so dass die Grundkörper (3) miteinander verbindbar, insbesondere gelenkig verbindbar, sind.

11. Haltevorrichtung nach einem der vorangegangenen Ansprüche, **dadurch gekennzeichnet, dass** der Grundkörper (3) Durchführkanäle (10) für elektrische Verbindungskabel (11) der Elektrodeneinrichtungen (2) besitzt.

12. Haltevorrichtung nach einem der vorangegangenen Ansprüche, **dadurch gekennzeichnet, dass** der Grundkörper (3) eine Durchtrittsöffnung (13) zur Bedienung von Bedienelementen der Elektrodeneinrichtungen (2) besitzt.

13. Datensatz, insbesondere auf einem Datenträger oder als von einem Datenträger herunterladbare Datei aufweisend 3D-Daten wenigstens der Haltevorrichtung (1) nach einem der Ansprüche 1 bis 12, welche 3D-druckbar sind oder in 3D-druckbare Daten umwandelbar sind.
